# EUROPEAN PATENT APPLICATION

(11) **EP 4 331 600 A1**
(43) Date of publication of application: **06.03.2024**
(21) Application number: 22794968.2
(22) Date of filing: 28.04.2022
(51) Int. Cl.: A61K 38/26, A61K 38/22, A61K 9/08, A61P 3/08, A61P 3/10, A61P 3/04

(54) **OXM3 STORAGE AGENT, OXM3 PREPARATION, AND PREPARATION METHOD**

(30) Priority: 30.04.2021 CN 202110485578; 20.04.2022 CN 202210420561
(71) Applicant: Innovent Biologics (Suzhou) Co., Ltd., Suzhou, Jiangsu 215123 (CN)
(72) Inventor: ZHAO, Chengcheng, Suzhou, Jiangsu 215123 (CN); WANG, Yinjue, Suzhou, Jiangsu 215123 (CN); WANG, Jingda, Suzhou, Jiangsu 215123 (CN)
(74) Representative: Nederlandsch Octrooibureau
(86) International application number: PCT/CN2022/089742
(87) International publication number: WO 2022/228498

(57) **Abstract**

Disclosed are an OXM3 storage agent, an OXM3 formulation and a preparation method. The OXM3 storage agent comprises 0.5-5 mg/mL tromethamine, 0.1-100 mg/mL stabilizer, 0.01-5 mg/mL chelating agent and a solvent, wherein: the stabilizer comprises one or more of mannitol, propylene glycol, arginine, arginine hydrochloride, histidine and histidine hydrochloride, the chelating agent comprises edetate disodium, and the solvent comprises water. The OXM3 formulation prepared from the OXM3 storage agent can ensure that the active ingredient OXM3 is stably stored for at least 6 months, preferably for 12 months or more, and more preferably for 18-24 months or more.

## Description

### CROSS-REFERENCE TO RELATED APPLICATIONS

The present invention claims priority to the invention patent application No. 202110485578.0 filed in China on April 30, 2021 and entitled "OXM3 STORAGE AGENT, OXM3 FORMULATION AND PREPARATION METHOD" and the invention patent application No. 202210420561.1 filed in China on April 20, 2022 and entitled "OXM3 STORAGE AGENT, OXM3 FORMULATION AND PREPARATION METHOD", the content of which is incorporated herein by reference in their entirety.

### TECHNICAL FIELD

The present invention belongs to the field of pharmaceutics, and particularly relates to an OXM3 storage agent, an OXM3 formulation and a preparation method.

### BACKGROUND

The stability of a drug is a key index for ensuring its efficacy and safety. A formula of formulation that imparts good stability to a drug is a key prerequisite to keep the efficacy and safety of a drug over the shelf life. Through the experiments on the forced stability, the accelerated stability, the shaking stability and the illumination stability, the influence of different auxiliary materials and inner packaging materials on the quality of the active ingredients is studied, and the formulas of the formulations are evaluated. The items tested during the study mainly include appearance, visible particles, purity (SEC-HPLC method and RP-HPLC method), charge variants (AEX method) and biological activity (cell-based method).

OXM3 is a dual agonist for glucagon-like peptide-1 (GLP-1) and glucagon receptors, and it can bind to and activate the glucagon-like peptide-1 receptor (GLP-1R) and the glucagon receptor (GCGR). It was first disclosed in CN201680036771.3. Its molecular formula is HXaa2QGTFTSDYSKYLDEKKAKEFVEWLLEGGPSSG, where the Xaa2 is Aib, the K at position 20 is chemically modified by conjugation to the ε-amino group of the K side chain with ([2-(2-amino-ethoxy)-ethoxy]-acetyl)2-(γGlu)1-CO-(CH2)18-CO2H, and the carboxyl group of the C-terminal glycine is amidated and becomes a C-terminal primary amide. The chemical formula of OXM3 is shown below.

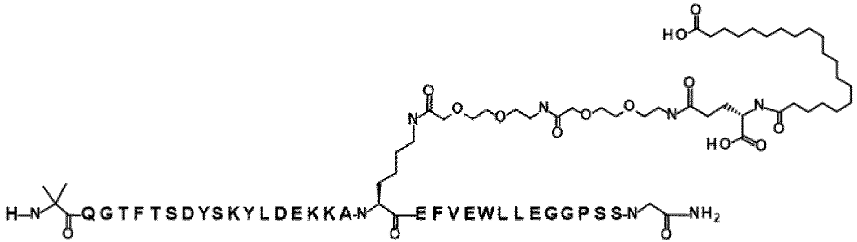

Since there is no liquid storage agent capable of well preserving OXM3 in the prior art, there is an urgent need for a storage agent capable of stably storing OXM3 and an OXM3 formulation capable of being stably stored.

### SUMMARY

Given that there is no liquid storage agent capable of well preserving OXM3 or an OXM3 formulation capable of being stably stored in the prior art, the present invention provides an OXM3 storage agent, an OXM3 formulation and a preparation method. The OXM3 formulation prepared from the OXM3 storage agent can ensure that the active ingredient OXM3 is stably stored for at least 6 months, preferably for 12 months or more, and more preferably for 18-24 months or more.

To solve the technical problem described above, the technical scheme I provided by the present invention is an OXM3 storage agent comprising 0.5-5 mg/mL tromethamine, 0.1-100 mg/mL stabilizer, 0.01-5 mg/mL chelating agent and a solvent, wherein: the stabilizer comprises one or more of mannitol, propylene glycol, arginine, arginine hydrochloride, histidine and histidine hydrochloride, and preferably the stabilizer comprises mannitol and propylene glycol; the chelating agent comprises edetate disodium; the solvent comprises water.

Preferably, the storage agent comprises 1-3 mg/mL tromethamine, 10-66 mg/mL stabilizer and 0.03-1 mg/mL chelating agent. Preferably, the storage agent comprises 1.21 mg/mL tromethamine, 20-46 mg/mL stabilizer and 0.05-0.5 mg/mL chelating agent.

In a preferred example of the technical scheme I, the storage agent consists of 1.21 mg/mL tromethamine, 46 mg/mL mannitol, 0.5 mg/mL edetate disodium and water as a solvent.

In a preferred example of the technical scheme I, the storage agent consists of 1.21 mg/mL tromethamine, 20 mg/mL propylene glycol, 0.5 mg/mL edetate disodium and water as a solvent.

In a preferred example of the technical scheme I, the storage agent consists of 1.21 mg/mL tromethamine, 10 mg/mL propylene glycol, 23 mg/mL mannitol, 0.05 mg/mL edetate disodium and water as a solvent.

Preferably, the storage agent further comprises a surfactant, preferably tween 80.

To solve the technical problem described above, the technical scheme II provided by the present invention is a method for preparing the OXM3 storage agent described above.

To solve the technical problem described above, the technical scheme III provided by the present invention is an OXM3 formulation comprising an OXM3 storage agent described above and 1-100 mg/mL OXM3, wherein the OXM3 has a concentration of, e.g., 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 18 mg/mL, 20 mg/mL, 30 mg/mL, 40 mg/mL, 50 mg/mL, 60 mg/mL, 70 mg/mL, 80 mg/mL, 90 mg/mL or 100 mg/mL. Preferably, the OXM3 has a concentration of 3-50 mg/mL. More preferably, the OXM3 has a concentration of 6-20 mg/mL; still preferably, the OXM3 has a concentration of 15-20 mg/mL; further preferably, the OXM3 has a concentration of 3 mg/mL, 4 mg/mL, 6 mg/mL, 8.37 mg/mL, 12 mg/mL, 15 mg/mL, 18 mg/mL or 20 mg/mL.

Preferably, the OXM3 formulation has a pH of 7-9. Preferably, the OXM3 formulation has a pH of 7.5-8.5. More preferably, the OXM3 formulation has a pH of 7.7.

In a preferred example of the technical scheme III, the OXM3 formulation consists of 8.37 mg/mL OXM3, 1.21 mg/mL tromethamine, 46 mg/mL mannitol, 0.5 mg/mL edetate disodium and water as a solvent, and the OXM3 formulation has a pH of 7.7.

In a preferred example of the technical scheme III, the OXM3 formulation consists of 8.37 mg/mL OXM3, 1.21 mg/mL tromethamine, 20 mg/mL propylene glycol, 0.5 mg/mL edetate disodium and water as a solvent, and the OXM3 formulation has a pH of 7.7.

In a preferred example of the technical scheme III, the OXM3 formulation consists of 3 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

In a preferred example of the technical scheme III, the OXM3 formulation consists of 4 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

In a preferred example of the technical scheme III, the OXM3 formulation consists of 6 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

In a preferred example of the technical scheme III, the OXM3 formulation consists of 12 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

In a preferred example of the technical scheme III, the OXM3 formulation consists of 15 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

In a preferred example of the technical scheme III, the OXM3 formulation consists of 18 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7. In a preferred example of the technical scheme III, the OXM3 formulation consists of 20 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

To solve the technical problem described above, the technical scheme IV provided by the present invention is a method for preparing the OXM3 formulation described above, which comprises the steps of:
(1) mixing tromethamine, a stabilizer and a chelating agent to obtain an OXM3 storage agent;
(2) mixing OXM3 and the OXM3 storage agent obtained in step (1) to obtain a mixed solution; and
(3) adjusting a pH of the mixed solution and filtering to obtain the OXM3 formulation.

To solve the technical problem described above, the technical scheme V provided by the present invention is use of an OXM3 storage agent described above in preserving OXM3.

To solve the technical problem described above, the technical scheme VI provided by the present invention is a liquid formulation prepared from the storage agent described above; the liquid formulation is preferably a water injection.

To solve the technical problem described above, the technical scheme VII provided by the present invention is a delivery device containing the OXM3 storage agent described above, the OXM3 formulation described above or the liquid formulation described above;
the delivery device preferably comprises a container, a seal and an injection needle;
wherein:
   the container is preferably a phial, a syringe or a vial;
   the seal is preferably a sealing plug or a sealing ring;
   the injection needle is preferably a water needle or a single needle-microneedle set.

In a preferred example of the technical scheme VII, the delivery device is a pre-filled syringe.

To solve the technical problem described above, the technical scheme VIII provided by the present invention is a kit comprising the OXM3 formulation described above, the liquid formulation described above or the delivery device described above.

To solve the technical problem described above, the technical scheme IX provided by the present invention is use of the OXM3 formulation described above, the liquid formulation described above, the delivery device described above or the kit described above in preparing a product for treating or preventing a disease in a subject, and the disease is preferably obesity, diabetes mellitus and non-alcoholic steatohepatitis.

The preferred conditions described above may be combined arbitrarily to obtain preferred embodiments of the present invention on the basis of the general knowledge in the art.

The reagents and starting materials used in the present invention are commercially available.

The beneficial effects of the present invention are as follows:
The present invention provides an OXM3 storage agent, an OXM3 formulation and a preparation method, wherein in the study for forced stability, accelerated stability and long-term stability, the OXM3 formulation prepared from the OXM3 storage agent meets the criteria for determining absence of quality change through the detection of such aspects as appearance, visible particles, content, purity, charge variants and biological activity and can be stably stored for at least 6 months and for at most 12 months or more. The storage, transportation and use of OXM3 are greatly facilitated.

### BRIEF DESCRIPTION OF THE DRAWINGS

FIG. 1 is a graph showing the trend of change in the monomer purity (SEC-HPLC method) (40±2 °C);
FIG. 2 is a graph showing the trend of change in the monomer purity (SEC-HPLC method) (25±2 °C);
FIG. 3 is a graph showing the trend of change in the main peak purity (RP-HPLC method) (40±2 °C);
FIG. 4 is a graph showing the trend of change in the main peak purity (RP-HPLC method) (25±2 °C);
FIG. 5 is a graph showing the trend of change in the charge variant-principal component (AEX method) (40±2 °C);
FIG. 6 is a graph showing the trend of change in the charge variant-acidic component (AEX method) (40±2 °C);
FIG. 7 is a graph showing the trend of change in the charge variant-principal component (AEX method) (25±2 °C);
FIG. 8 is a graph showing the trend of change in the charge variant-acidic component (AEX method) (25±2 °C);
FIG. 9 is a graph showing the trend of change in the main peak purity (RP-HPLC method) (40±2 °C);
FIG. 10 is a graph showing the trend of change in the main peak purity (RP-HPLC method) (25±2 °C);
FIG. 11 is a graph showing the trend of change in the total impurities (RP-HPLC method) (40±2 °C);
FIG. 12 is a graph showing the trend of change in the total impurities (RP-HPLC method) (25±2 °C);
FIG. 13 is a graph showing the trend of change in the charge variant-principal component (AEX method) (40±2 °C);
FIG. 14 is a graph showing the trend of change in the charge variant-acidic component (AEX method) (40±2 °C);
FIG. 15 is a graph showing the trend of change in the charge variant-principal component (AEX method) (25±2 °C); and
FIG. 16 is a graph showing the trend of change in the charge variant-acidic component (AEX method) (25±2 °C).

### DETAILED DESCRIPTION

The present invention is further illustrated by the following examples; however, these examples should not be construed as limiting the present invention. Experimental procedures without specified conditions in the following examples are conducted in accordance with conventional procedures and conditions, or in accordance with the manufacturer's manual.

### 1. Experimental materials and instruments

### 1.1 Experimental samples

OXM3: content coefficient 0.837, purchased from Eli Lily, batch No. BO1706P002; or
OXM3: purchased from Asymchem, batch No. CPo125104-01-03-02-41-01.

### 1.2 Experimental materials, see Table 1.

**Table 1. Experimental materials**

| Name | Grade | Manufacturer & brand | Cat# | Criteria |
|---|---|---|---|---|
| Tromethamine (Tris) | Pharmaceutical grade | Merck, Germany | 1.80386.1000 | *Ch.P* (2015 Edition) |
| Mannitol | Pharmaceutical grade | Roquette, French | H20120407 | *Ch.P* (2015 Edition) |
| Propylene glycol | Pharmaceutical grade | Well, Nanjing | Jiangsu MPA Drug Approval No. F15214403 | *Ch.P* (2015 Edition) |
| Edetate disodium | Pharmaceutical grade | Nanjing Chemical Reagent Co., Ltd. | Jiangsu MPA Drug Approval No. F15431201 | *Ch.P* (2015 Edition) |
| Edetate disodium | Pharmaceutical grade | Avantor, USA | 8995-01 | USP |
| Diluted hydrochloric acid | Pharmaceutical grade | Hunan Er-Kang Pharmaceutical Co., Ltd. | NMPA Drug Approval No. H42020202 | *Ch.P* (2015 Edition) |
| Sodium hydroxide | Pharmaceutical grade | Jinshan Pharmaceutical | Sichuan MPA Drug Approval No. F20100001 | *Ch.P* (2015 Edition) |
| Sodium hydroxide | Pharmaceutical grade | Hunan Er-Kang Pharmaceutical Co., Ltd. | Human MPA Food and Pharmaceutical Auxiliary Material Approval No. F20050011 | *Ch.P* (2015 Edition) |
| Needle-type filter | N/A | Merck Millipore, Germany | SLGV013SL | N/A |
| 2R vial | N/A | Schott, Suzhou | 1142144 | N/A |
| 13 mm rubber stopper | N/A | West, Singapore | 7002-4376 | N/A |
| 13 mm aluminum-plastic cap | N/A | West, Singapore | 5413-1065 | N/A |
| 1 mL pre-filled syringe | N/A | BD, France | 47363110 | N/A |
| Rubber stopper for BD pre-filled syringe | N/A | BD, France | 47375910 | N/A |
| AI auto injector assembly | N/A | Ypsomed AG, Switzerland | N/A | N/A |

| | | | | |
|---|---|---|---|---|
| Note: N/A denotes not applicable | | | | |

### 1.3 Instruments, see Table 2.

### Table 2. Instruments

**Table 2. Criteria for determining absence of quality change**

| Name | Manufacturer & brand | Model No. | Device No. |
|---|---|---|---|
| Electronic balance | Sartorius, Germany | BSA224S | PD-A1-186 |
| pH meter | Mettler, Switzerland | S220 | PD-A 1-246 |
| Multi-parameter tester | Mettler, Switzerland | S220 | AS-A1-029 |
| Biochemical incubator | Jinghong, Shanghai | SHP-150 | PD-A 1-200 |
| Ultra-low temperature freezer | Thermo, USA | 907 | MFG-M1-211 |
| Multi-channel microspectrophotometer | Thermo, USA | nanodrop8000 | PD-A1-052 |
| Clarity detector | Tianda Tianfa, Tianjin | YB-2 | PD-A1-033 |
| Freezing point osmometer | Gonotec, Germany | OSMOMAT 3000D | AS-A1-131 |
| Liquid particle counting system | Beckman Coulter, USA | 9703+ | AS-A1-166 |

According to the knowledge of the product and the precision of the instrument and the method, criteria for determining absence of quality change in sample test indexes as compared to initial values were set, so as to determine whether the sample changed, as detailed in Table 3.

**Table 3. Criteria for determining absence of quality change**

| Test items | Criteria for determining absence of change |
|---|---|
| Appearance (visual | Clear and colorless liquid, no particles |
| inspection) | |
| Content (RP-HPLC method) | Change rate ≤ 10% |
| Visible particles (test for visible particles) | Conforms to the General Rule 0904 of the *Pharmacopoeia of the People's Republic of China* (2020 edition, volume III) |
| Monomer purity (SEC-HPLC method) | Main peak change ≤ 1.0% |
| Main peak purity (RP-HPLC method) | Main peak change ≤ 2.0% |
| Charge variants (AEX method) | Changes in principal component, acidic component and basic component ≤ 2.0% |
| Biological activity (cell-based method) | Should be 60-140% |

Specific methods for testing items in Table 3 are as follows:
Appearance: the appearance of the product was visually inspected.
Content: RP-HPLC method: a reversed-phase chromatographic column was used, the mobile phase A was 0.1% (v/v) TFA aqueous solution, and the mobile phase B was 0.085% (v/v) TFA acetonitrile solution; the diluent was prepared by well mixing 980 µL of ultra-pure water and 20 µL of 1 mol/L Tris-HCl buffer with a pH of 8.0, and the diluent was freshly prepared; the control solution was 1 vial of the control IBI362, which was slightly inverted for mixing; the sensitivity solution was prepared by pipetting 50 µL of the control IBI362 into a 10 mL volumetric flask and adding 20 mmol/L Tris buffer to bring the volume to 10 mL; the test sample was diluted to 1.0 mg/mL with ultra-pure water to serve as the sample solution; chromatographic conditions were as follows: the detection wavelength was 220 nm, the column temperature was 60 °C, the column pressure was 400 bar, the sample tray temperature was 5 °C, the flow rate was 1.2 mL/min, the sample introduction volume was 10 µL, the collection time was 60 min, and the elution gradient was as follows:

| Time (min) | Mobile phase A (%) | Mobile phase B (%) |
|---|---|---|
| 0 | 90.0 | 10.0 |
| 20.0 | 55.0 | 45.0 |
| 52.0 | 47.0 | 53.0 |
| 53.0 | 10.0 | 90.0 |
| 55.0 | 10.0 | 90.0 |
| 55.1 | 90.0 | 10.0 |
| 60.0 | 90.0 | 10.0 |

Precisely 10 µL of each of the diluent, the control solution, the sensitivity solution and the sample solution was pipetted and introduced for testing.

Visible particles: the visible particles in the sample were detected using a clarity detector (model No. YB-2, Tianda Tianfa, Tianjin) and an insoluble particle detector (model No. GWJ-8, Tianda Tianfa, Tianjin) according to the method described in the National Pharmacopoeia Committee, the *Pharmacopoeia of the People's Republic of China* (2015 edition, volume IV General Rules 0904 "Test for Visible Particles", Beijing, China Medical Science Press, 2015).

Monomer purity: SEC-HPLC method: the separation was performed on a size exclusion chromatographic column, and the mobile phase was 80% 20 mmol/L Tris buffer+20% acetonitrile; the diluent was prepared by well mixing 980 µL of ultra-pure water and 20 µL of 1 mol/L Tris-HCl buffer with a pH of 8.0, and the diluent was freshly prepared; the control solution was 1 vial of the control IBI362, which was slightly inverted for mixing; the sensitivity solution was prepared by pipetting 50 µL of the control IBI362 into a 10 mL volumetric flask and adding 20 mmol/L Tris buffer to bring the volume to 10 mL; the test sample was diluted to 1.0 mg/mL with ultra-pure water to serve as the sample solution; chromatographic conditions were as follows: the sample introduction volume was 10 µL, the flow rate was 0.5 mL/min, the collection time was 35 min, the column temperature was 25 °C, the column pressure was 1000 psi/70 bar, the sample tray temperature was 6 °C, and the detection wavelength was 214 nm. The elution mode was isocratic elution. Precisely 10 µL of each of the diluent, the control solution, the sensitivity solution and the sample solution was pipetted and introduced for testing.

Main peak purity: RP-HPLC method, same as the determination method for content.

Charge variants: anion exchange chromatography (AEX-HPLC method combined with UV detection). Separation was performed using YMC-Biopro QA-F (100×4.6 mm, S-5 µm). The mobile phase A was 100 mM sodium acetate (pH 4.6) and 50% acetonitrile (8.20 g of sodium acetate was added to 400 mL of water in a 1000 mL graduated cylinder, the pH was adjusted to 4.6 with acetic acid, water was added to reach a total volume of 500 mL, and then the volume was brought to 1000 mL with acetonitrile), and the mobile phase B was 50% acetonitrile/water (500 mL acetonitrile was added to a 1000 mL graduated cylinder and then the volume was brought to 1000 mL with water). The 1 mg/mL solution was prepared with 20 mM TRIS buffer with a pH of 8.0 (2.42 g of TRIS was dissolved in 1000 mL of water, and the pH was adjusted to 8.0±0.1 with 1.0 N HCl). 20 mM TRIS buffer was used as the blank solution. As a detection standard, a reference standard was used (concentration: 1.0 mg/mL). 10 µL of each of the blank solution, the detection standard solution and the sample solution was injected into the liquid chromatograph. The flow rate of the mobile phase was 1.0 mL/min, the collection time was 45 min, the column temperature was 25 °C, the detection wavelength was 280 nm, and the temperature of the autosampler was 2-8 °C. The solution injected was analyzed, and the content of the principal component, the acidic component and the basic component was calculated.

Biological activity: (including assays for GLP-1 activity and glucagon activity)
1) GLP-1 activity: the old medium for cultured HEK293-GLP1R cells was pipetted out, the cells were washed once with 5 mL of PBS and then digested with 1 mL of Accutase solution until the cells fell off, and then Assay Buffer (10% FBS (56 mL), 90% DMEM (1×) + GlutaMAXTM-l medium (500 mL)) was added to terminate the digestion. The cell suspension was collected and centrifuged for 5 min at 1000 r/min, the supernatant was discarded, the cells were counted after being resuspended in Assay Buffer, and then the density of the cell suspension was adjusted to 0.2×10⁶ cells/mL. The control and the sample were each subjected to three-fold serial dilution for 10 gradients with Assay Buffer from an initial concentration of 534 ng/mL, and samples of 10 gradients were obtained. The processed sample and the cell suspension were added into a 96-well white cell culture plate at 50 µL/well according to the experimental arrangement; for the negative control group, Assay Buffer was added at 50 µL/well, and the peripheral wells were sealed with Assay Buffer at 100 µL/well. Then the mixture was incubated in an incubator at 37±2 °C and 5%±1% COz for 6 h. The prepared chromogenic solution (obtained by adding 100 mL of Bio-GloTM Luciferase Assay buffer into Bio-GloTM Luciferase Assay Substrate for dissolution) was equilibrated to room temperature in advance. The cell culture plate was taken out and left to stand for 10 min to equilibrate to room temperature. The chromogenic solution was added at 100 µL/well, and the resulting mixture was left to stand at room temperature for 5 min. The full-wavelength chemiluminescence values were read by using a microplate reader, and the four-parameter curve fitting was performed with the protein concentration as the abscissa and the induction fold as the ordinate to obtain data such as EC₅₀ and R². Biological activity% = EC₅₀ of control/EC₅₀ of sample × 100%;
2) Glucagon activity: the old medium for cultured CHO-K1/GCGR/Ga15 cells was pipetted out, the cells were washed once with 1× PBS and then digested with 1 mL of Accutase solution for 4-5 min in an incubator at 37±2 °C and 5%±1% COz, and then F-12 (1×) was added to terminate the digestion. The cells were centrifuged for 5 min at 1000 r/min, the supernatant was discarded, the cells were counted after being resuspended in a proper amount of assay medium, and then the density of the cell suspension was adjusted to 1.8×10⁶ cells/mL. The control and the sample were each subjected to three-fold serial dilution for 10 gradients with the sample diluent (20% Stimulation Buffer, 80% sterile water (freshly prepared as needed)) from an initial concentration of 267 ng/mL, and samples of 10 gradients were obtained. The processed sample and the cell suspension were added into a 96-well plate at 5 µL/well according to the predetermined arrangement, then the transient centrifugation was performed, and then the plate was sealed with a film and incubated for 30 min in an incubator at 37±2 °C and 5%±1% COz. The 96-well plate was taken out, the film was removed, and the plate was subjected to transient centrifugation. The prepared cAMP-d2 stock solution and Anti-cAMP-Cryptate stock solution were thawed in advance and equilibrated to room temperature to prepare the working solution. 5 µL of cAMP-d2 working solution (20% cAMP-d2 stock solution + 80% Lysis&Detection Buffer (freshly prepared as needed)) and 5 µL of Anti-cAMP-Cryptate working solution (20% Anti-cAMP-Cryptate stock solution + 80% Lysis&Detection Buffer (freshly prepared as needed)) were added to each well, the transient centrifugation was performed, and then the mixture was subjected to color development at room temperature in the dark for 60 min. The full-wavelength chemiluminescence values were read by using a microplate reader, and the four-parameter curve fitting was performed with the protein concentration as the abscissa and the Ratio value as the ordinate to obtain data such as EC₅₀ and R². Biological activity% = EC₅₀ of control/EC₅₀ of sample × 100%.

### Example 1: Preparation of OXM3 Formulations Based on Formulas F1-F4

Buffers for the formulas were prepared according to Table 4, and OXM3 was added to each formula solution according to corresponding amount specified in the formula. The pH was adjusted to 7.7. The solutions were each filtered and placed into 2R vials at 1 mL/vial, followed by plugging and capping.

**Table 4. Information about formulas**

| No. | Composition | | | | | |
|---|---|---|---|---|---|---|
| | OXM3 (mg/ml) | Tromethamine (mg/mL) | Mannitol (mg/mL) | Propylene glycol (mg/mL) | Edetate disodium (mg/mL) | pH value |
| F1 | 8.37 | 1.21 | 46 | / | / | 7.7 |
| F2 | 8.37 | 1.21 | / | 20 | / | 7.7 |
| F3 | 8.37 | 1.21 | 46 | / | 0.5 | 7.7 |
| F4 | 8.37 | 1.21 | / | 20 | 0.5 | 7.7 |

| | | | | | | |
|---|---|---|---|---|---|---|
| Note: the solvent of the formulations described above is water for injection; "/" indicates that this auxiliary material is not added. | | | | | | |

### Example 2: Experiment on Stability of Formulas F1-F4

Samples based on the formulas described above were subjected to study for forced stability, accelerated stability and long-term stability. The specific scheme is shown in Table 5.

**Table 5. Experimental conditions and sampling schedule**

| Name of experiment | Formula | Experimental conditions and sampling schedule | Test items |
|---|---|---|---|
| Experiment on forced stability | F1∼F4 | Stored at 40±2 °C, sampling at day 0, week 1, week 2 and month 1 | Appearance, visible particles, content (reversed-phase high performance liquid chromatography (RP-HPLC)), purity (size exclusion chromatography-high performance liquid chromatography (SEC-HPLC) and RP-HPLC method), charge variants (anion exchange chromatography (AEX)) and biological activity (cell-based method) |
| Experiment on accelerated stability | F1∼F4 | Stored at 25±2 °C, sampling at day 0, week 2, month 1 and month 3 | |
| Experiment on long-term stability | F1∼F4 | Stored at 5±3 °C, sampling at day 0 and month 3 | |

### Analysis of results:

### (1) Appearance and visible particles

F1-F4 samples were acceptable in terms of appearance and visible particles after storage at 40±2 °C for 1 month, at 25±2 °C for 3 months and at 5±3 °C for 3 months.

### (2) Content

The results of drug content are shown in Table 6. The results show that after storage at 40±2 °C for 1 month, the drug content values of the F1 and F2 samples were significantly decreased; compared with values on day 0, the drug content values of the F1 and F2 samples were decreased by 1.1 mg/mL and 1.0 mg/mL, respectively, while the drug content values of the F3 and F4 samples were only decreased by 0.2 mg/mL and 0.4 mg/mL, respectively, and showed no significant changes. Similarly, after storage at 25±2 °C for 3 months, the drug content values of the F1 and F2 samples were significantly decreased; compared with values on day 0, the drug content values of the F1 and F2 samples were decreased by 1.1 mg/mL and 0.9 mg/mL, respectively, while the drug content values of the F3 and F4 samples were only decreased by 0.2 mg/mL and 0.3 mg/mL, respectively, and showed no significant changes. The drug content value of each sample showed no significant change after storage at 5±3 °C for 3 months. In summary, OXM3 is stable in F3 and F4.

**Table 6. Results of drug content (RP-HPLC method, mg/mL)**

| Sample name | Day 0 | 40 °C±2 °C | | | 25 °C±2 °C | | | 5 °C±3 °C |
|---|---|---|---|---|---|---|---|---|
| | | 1 week | 2 weeks | 1 month | 2 weeks | 1 month | 3 months | 3 months |
| F1 | 7.9 | 7.7 | 7.5 | 6.8 | 7.8 | 7.3 | 6.8 | 7.8 |
| F2 | 8.2 | 8.1 | 7.9 | 7.2 | 8.2 | 7.6 | 7.3 | 8.1 |
| F3 | 7.8 | 7.9 | 7.8 | 7.6 | 7.9 | 7.7 | 7.6 | 8.0 |
| F4 | 8.0 | 8.1 | 8.1 | 7.6 | 8.2 | 7.9 | 7.7 | 8.1 |

### (3) Purity

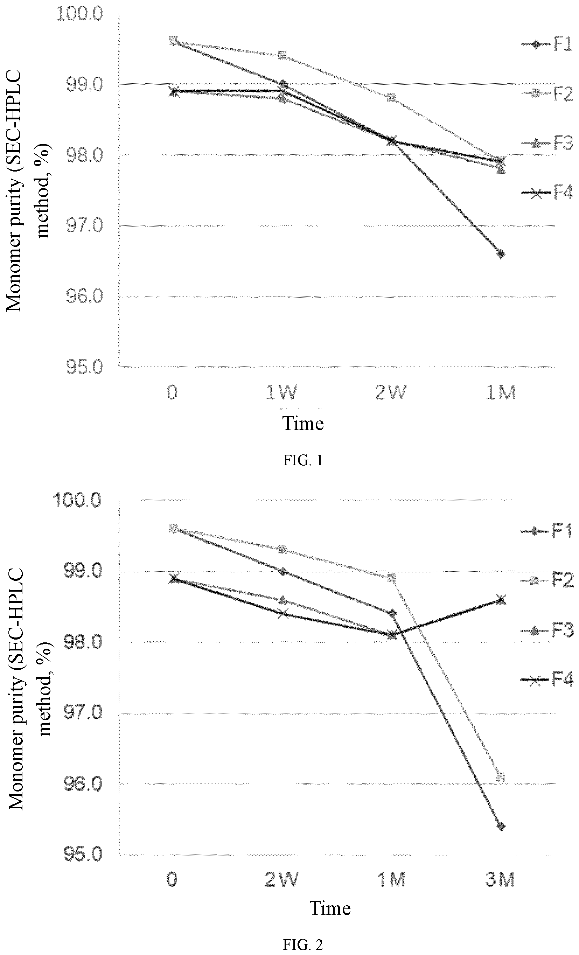

The results of purity are shown in Table 7. The trend of change in the monomer purity (SEC-HPLC method) is shown in FIG. 1 and FIG. 2. The results show that after storage at 40±2 °C for 1 month, the monomer purity values of the F1-F4 samples were all significantly decreased; compared with values on day 0, the monomer purity values of the samples were decreased by 3.0%, 1.7%, 1.1% and 1.0%, respectively, and the decrease of F3 and F4 was significantly smaller than that of F1 and F2. After storage at 25±2 °C for 3 months, the monomer purity values of F1 and F2 (SEC-HPLC method) were decreased by 4.2% and 3.5%, respectively, which failed to meet the criteria for determining absence of change, while the monomer purity values of both the F3 and F4 were decreased by 0.3% and showed no significant changes. The monomer purity values of F1-F4 all showed no significant changes after storage at 5±3 °C for 3 months.

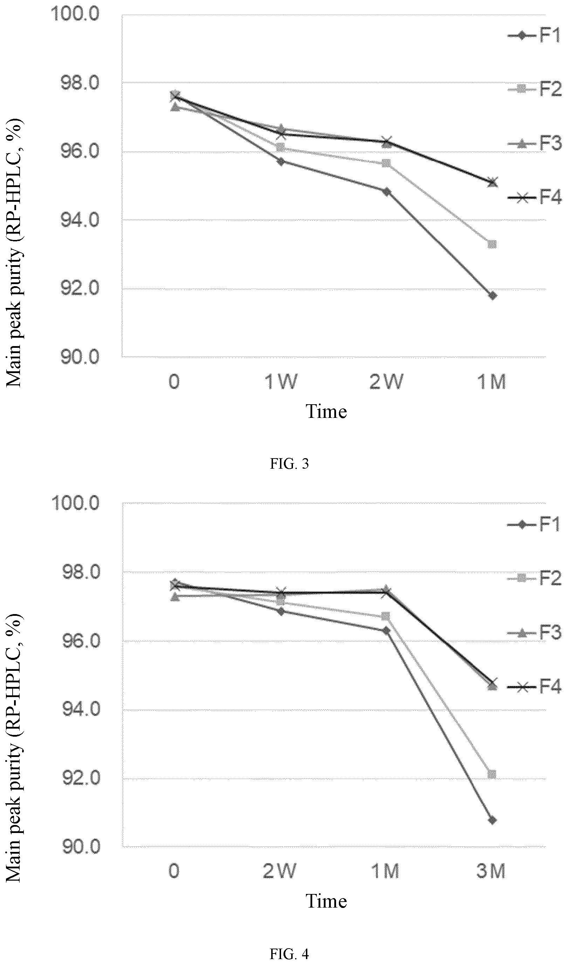

The trend of change in the main peak purity (RP-HPLC method) is shown in FIG. 3 and FIG. 4. After storage at 40±2 °C for 1 month, the main peak purity values of the F1-F4 samples were all significantly decreased; compared with values on day 0, the main peak purity values of the F1-F4 samples were decreased by 5.9%, 4.3%, 2.2% and 2.5%, respectively, and the decrease of F3 and F4 was significantly smaller than that of F1 and F2. After storage at 25±2 °C for 3 months, the main peak purity values of F1 and F2 were decreased by 6.9% and 5.5%, respectively, the main peak purity values of F3 and F4 were decreased by 2.6% and 2.8%, respectively, and the decrease of F3 and F4 was significantly smaller than that of F1 and F2. The monomer purity values of F1-F4 all showed no significant changes after storage at 5±3 °C for 3 months.

In summary, F3 and F4 are better than F1 and F2.

**Table 7. Results of purity**

| Test items | Sample name | Day 0 | 40 °C±2 °C | | | 25 °C±2 °C | | | 5 °C±3 °C |
|---|---|---|---|---|---|---|---|---|---|
| | | | 1 week | 2 weeks | 1 month | 2 weeks | 1 month | 3 months | 3 months |
| Purity (SEC-HPLC method, %) | F1 | 99.6 | 99.0 | 98.2 | 96.6 | 99.0 | 98.4 | 95.4 | 99.3 |
| | F2 | 99.6 | 99.4 | 98.8 | 97.9 | 99.3 | 98.9 | 96.1 | 99.4 |
| | F3 | 98.9 | 98.8 | 98.2 | 97.8 | 98.6 | 98.1 | 98.6 | 98.8 |
| | F4 | 98.9 | 98.9 | 98.2 | 97.9 | 98.4 | 98.1 | 98.6 | 98.9 |
| Purity (RP-HPLC method, %) | F1 | 97.7 | 95.7 | 94.8 | 91.8 | 96.9 | 96.3 | 90.8 | 97.5 |
| | F2 | 97.6 | 96.1 | 95.6 | 93.3 | 97.1 | 96.7 | 92.1 | 97.7 |
| | F3 | 97.3 | 96.7 | 96.3 | 95.1 | 97.4 | 97.5 | 94.7 | 97.8 |
| | F4 | 97.6 | 96.5 | 96.3 | 95.1 | 97.4 | 97.4 | 94.8 | 97.7 |

### (4) Charge variants

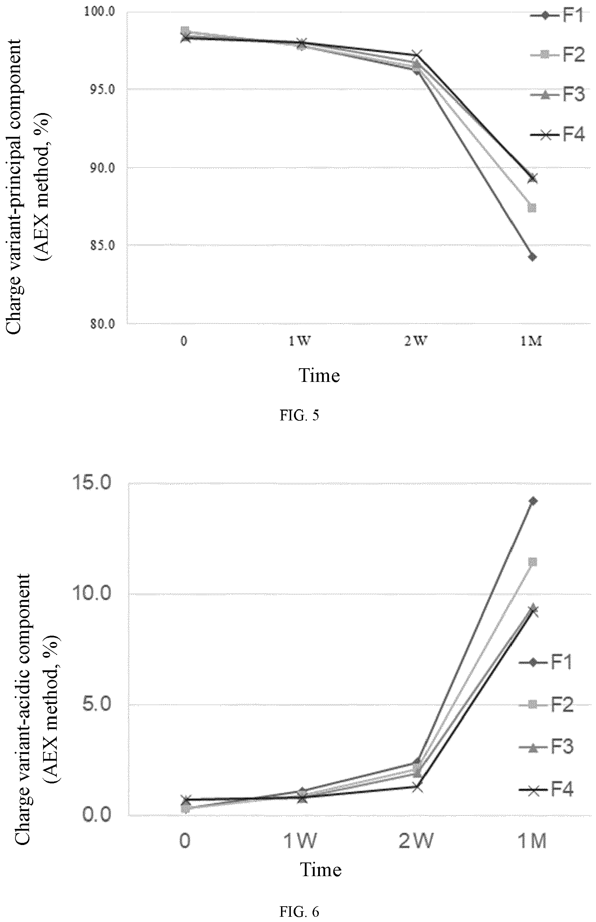

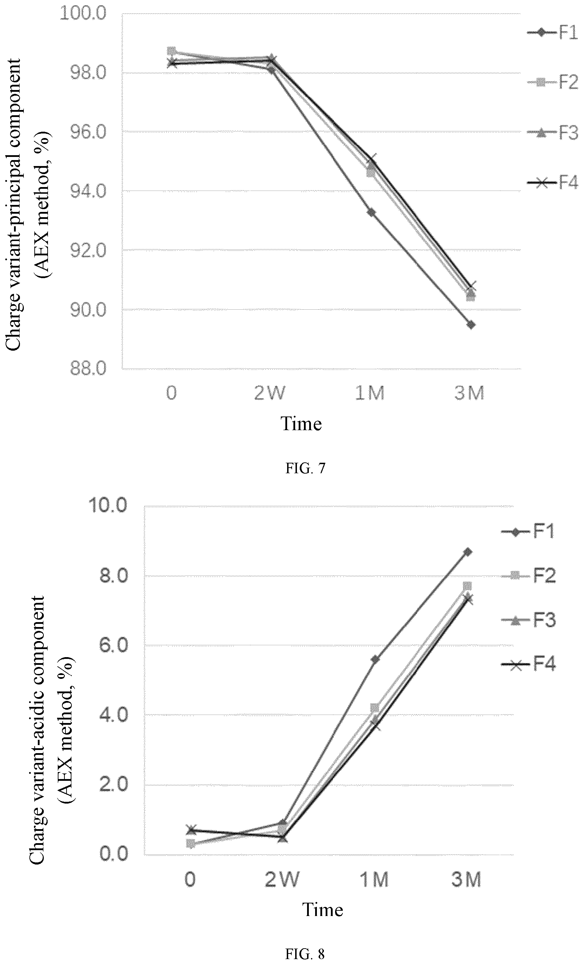

The results of charge variants are shown in Table 8. The trend of change is shown in FIGs. 5-8.

The results show that after storage at 40±2 °C for 1 month, the principal component and the acidic component of the charge variants of all formula samples showed significant changes; compared with values on day 0, the principal component values of the F1-F4 samples were decreased by 14.4%, 11.3%, 9.0% and 9.0%, respectively, the acidic component values were increased by 13.9%, 11.1%, 8.7% and 8.5%, respectively, and the changes of F3 and F4 were significantly smaller than those of F1 and F2. After storage at 25±2 °C for 3 months, the principal component and the acidic component of the charge variants of all formula samples showed significant changes; compared with values on day 0, the principal component values of the F1-F4 samples were decreased by 9.2%, 8.3%, 7.8% and 7.5%, respectively, the acidic component values were increased by 8.4%, 7.4%, 6.7% and 6.6%, respectively, and the changes of F3 and F4 were smaller than those of F1 and F2. After storage at 5±3 °C for 3 months, the principal component and the acidic component of the charge variants of all formula samples showed no significant changes. In summary, F3 and F4 are better than F1 and F2.

**Table 8. Results of charge variants (AEX method, %)**

| Sample name | | Day 0 | 40 °C±2 °C | | | 25 °C±2 °C | | | 5 °C±3 °C |
|---|---|---|---|---|---|---|---|---|---|
| | | | 1 week | 2 weeks | 1 month | 2 weeks | 1 month | 3 months | 3 months |
| F1 | Acidic component | 0.3 | 1.1 | 2.4 | 14.2 | 0.9 | 5.6 | 8.7 | 0.5 |
| | Principal component | 98.7 | 97.8 | 96.2 | 84.3 | 98.1 | 93.3 | 89.5 | 98.4 |
| | Basic component | 1.0 | 1.1 | 1.4 | 1.5 | 1.0 | 1.2 | 1.8 | 1.1 |
| F2 | Acidic component | 0.3 | 0.9 | 2.1 | 11.4 | 0.7 | 4.2 | 7.7 | 0.4 |
| | Principal component | 98.7 | 97.8 | 96.4 | 87.4 | 98.3 | 94.6 | 90.4 | 98.6 |
| | Basic component | 1.0 | 1.3 | 1.5 | 1.2 | 1.1 | 1.2 | 1.8 | 1.0 |
| F3 | Acidic component | 0.7 | 0.8 | 1.9 | 9.4 | 0.5 | 3.9 | 7.4 | 0.5 |
| | Principal component | 98.4 | 98.0 | 96.7 | 89.4 | 98.5 | 94.9 | 90.6 | 98.5 |
| | Basic component | 0.9 | 1.2 | 1.4 | 1.2 | 1.1 | 1.2 | 2.0 | 1.0 |
| F4 | Acidic component | 0.7 | 0.8 | 1.3 | 9.2 | 0.5 | 3.7 | 7.3 | 0.5 |
| | Principal component | 98.3 | 98.0 | 97.2 | 89.3 | 98.4 | 95.1 | 90.8 | 98.4 |
| | Basic component | 1.0 | 1.2 | 1.4 | 1.4 | 1.1 | 1.2 | 2.0 | 1.0 |

### (5) Biological activity (cell-based method)

The results are shown in Table 9. The results show that the biological activity (cell-based method) of the F1-F4 samples was acceptable within the range of 60-140% after storage at 40±2 °C for 1 month, at 25±2 °C for 3 months and at 5±3 °C for 3 months.

**Table 9. Results of biological activity (cell-based method)**

| Test items | Sample name | Day 0 | 40 °C±2 °C | 25 °C±2 °C | 5 °C±3 °C |
|---|---|---|---|---|---|
| | | | 1 month | 3 months | 3 months |
| GLP-1 activity (cell-based method, %) | F1 | 93 | 118 | 89 | 113 |
| | F2 | 95 | 118 | 95 | 120 |
| | F3 | 110 | 105 | 109 | 89 |
| | F4 | 114 | 101 | 114 | 101 |
| Glucagon activity (cell-based method, %) | F1 | 114 | 75 | 133 | 85 |
| | F2 | 78 | 87 | 90 | 104 |
| | F3 | 83 | 124 | 100 | 110 |
| | F4 | 120 | 103 | 134 | 89 |

### Conclusion:

In summary, F3 and F4 are better than F1 and F2, which indicates that the effect of adding the edetate disodium as a stabilizer is significant; while there was no significant difference between F3 and F4, indicating that mannitol and propylene glycol have no significant effect on sample stability.

### Example 3: Preparation of OXM3 Formulations Based on Optimized Formula F5

Based on the results of the above examples and experience in developing formulas of formulations, the formula was optimized as follows: 3.0, 4.0, 6.0, 12.0, 15.0, 18.0, 20.0 mg/mL OXM3, 1.21 mg/mL tromethamine (i.e., tris(hydroxymethyl)aminomethane, Tris), 23.0 mg/mL mannitol, 10.0 mg/mL propylene glycol, 0.05 mg/mL edetate disodium, pH 7.7; the mixtures were each placed into 1 mL slender pre-filled syringes or 2R vials at an amount of 0.5 mL to obtain formulas F5-1, F5-2, F5-3, F5-4, F5-5, F5-6 and F5-7. The information about formulas is shown in Table 10.

**Table 10. Information about formulas**

| No. | Composition | | | | | |
|---|---|---|---|---|---|---|
| | OXM3 (mg/ml) | Tromethamine (mg/mL) | Mannitol (mg/mL) | Propylene glycol (mg/mL) | Edetate disodium (mg/mL) | pH value |
| F5-1 | 3.0 | 1.21 | 23 | 10 | 0.05 | 7.7 |
| F5-2 | 4.0 | 1.21 | 23 | 10 | 0.05 | 7.7 |
| F5-3 | 6.0 | 1.21 | 23 | 10 | 0.05 | 7.7 |
| F5-4 | 12.0 | 1.21 | 23 | 10 | 0.05 | 7.7 |
| F5-5 | 15.0 | 1.21 | 23 | 10 | 0.05 | 7.7 |
| F5-6 | 18.0 | 1.21 | 23 | 10 | 0.05 | 7.7 |
| F5-7 | 20.0 | 1.21 | 23 | 10 | 0.05 | 7.7 |

| | | | | | | |
|---|---|---|---|---|---|---|
| Note: the solvent of the formulations described above is water for injection. | | | | | | |

### Example 4: Experiment on Stability of Formula F5

I. The F5-1 and F5-2 samples prepared in Example 3 were subjected to study for forced stability, accelerated stability and long-term stability. The specific scheme is shown in Table 11.

**Table 11. Experimental conditions and sampling schedule**

| Name of experiment | Sample | Experimental conditions and sampling schedule | Test items |
|---|---|---|---|
| Experiment on forced stability | F5-1 and F5-2 | Stored at 40±2 °C, sampling at day 0, week 2 and week 4 | Appearance, visible particles, content (RP-HPLC method), insoluble particles (light blockage), purity (SEC-HPLC method and RP-HPLC method) and biological activity (cell-based method) |
| Experiment on accelerated stability | | Stored at 25±2 °C, sampling at day 0, month 1, month 2 and month 3 | |
| Experiment on long-term stability | | Stored at 5±3 °C, sampling at day 0, month 3 and month 6 | |

### Analysis of results

The data in Tables 12-13 show that after storage at 40±2 °C for 4 weeks, the F5-1 and F5-2 samples showed no significant changes in test items of appearance, activity and visible particles; they showed changes in test items of content, purity and related substances, but the changes were acceptable in terms of the quality standards.

**Table 12. Data on forced stability of F5-1 (40±2 °C)**

| Test items | Acceptance criteria | Study time | | |
|---|---|---|---|---|
| | | Day 0 | 2 weeks | 4 weeks |
| Appearance (visual inspection) | Should be clear to slightly opalescent, colorless or nearly colorless liquid | Clear, colorless liquid | Clear, colorless liquid | Clear, colorless liquid |
| GLP-1 activity (cell bioassay) | Should be 60-140% of the control | 113 | 92 | 133 |
| Glucagon activity (cell bioassay) | Should be 60-140% of the control | 71 | 77 | 77 |
| Main peak purity (RP-HPLC method) | Should be ≥ 92.0% | 97.9 | 95.8 | 93.1 |
| Related substances (RP-HPLC method) | Maximum unspecific impurities: should be ≤ 4.0% | 0.6 | 0.7 | 1.7 |
| | Total impurities: should be ≤ 8.0% | 2.1 | 4.2 | 6.9 |
| monomer/polymer (SEC-HPLC method) | Monomer: should be ≥ 97.0% | 99.4 | 99.2 | 99.0 |
| | Polymer: should be ≤ 3.0% | 0.3 | 0.5 | 0.8 |
| Visible particles (test for visible particles) | Should comply with specification | Comply with specification | N/A | N/A |
| pH value (method for determining pH) | Should be 7.5-8.5 | 7.7 | 7.7 | 7.7 |
| Insoluble particles (light blockage) | The number of particles with the diameter ≥ 10 µm in each vial should be ≤ 6000 | 1278 | N/A | N/A |
| | The number of particles with the diameter ≥ 25 µm in each vial should be ≤ 600 | 6 | N/A | N/A |
| Content determination (RP-HPLC method) | Should be 90.0-110.0% of the labeled amount | 96.5 | 94.5 | 91.0 |

**Table 13. Data on forced stability of F5-2 (40±2 °C)**

| Test items | Acceptance criteria | Study time | | |
|---|---|---|---|---|
| | | Day 0 | 2 weeks | 4 weeks |
| Appearance (visual inspection) | Should be clear to slightly opalescent, colorless or nearly colorless liquid | Clear, colorless liquid | Clear, colorless liquid | Clear, colorless liquid |
| GLP-1 activity (cell bioassay) | Should be 60-140% of the control | 96 | 99 | 99 |
| Glucagon activity (cell bioassay) | Should be 60-140% of the control | 93 | 73 | 86 |
| Main peak purity (RP-HPLC method) | Should be ≥ 92.0% | 98.0 | 95.9 | 93.4 |
| Related substances (RP-HPLC method) | Maximum unspecific impurities: should be ≤ 4.0% | 0.6 | 0.7 | 1.6 |
| | Total impurities: should be ≤ 8.0% | 2.0 | 4.1 | 6.6 |
| Monomer/polymer (SEC-HPLC method) | Monomer: should be ≥ 97.0% | 99.5 | 99.3 | 99.1 |
| | Polymer: should be ≤ 3.0% | 0.3 | 0.6 | 0.8 |
| Visible particles (test for visible particles) | Should comply with specification | Comply with specification | N/A | N/A |
| pH value (method for determining pH) | Should be 7.5-8.5 | 7.7 | 7.7 | 7.7 |
| Insoluble particles (light blockage) | The number of particles with the diameter ≥ 10 µm in each vial should be ≤ 6000 | 1312 | N/A | N/A |
| | The number of particles with the diameter ≥ 25 µm in each vial should be ≤ 600 | 11 | N/A | N/A |
| Content determination (RP-HPLC method) | Should be 90.0-110.0% of the labeled amount | 97.3 | 95.8 | 91.0 |

The data in Tables 14-15 show that after storage at 25±2 °C for 3 months, the F5-1 and F5-2 samples showed no significant changes in test items of appearance, activity and visible particles; they showed changes in test items of content, purity and related substances, but the changes were acceptable in terms of the quality standards.

**Table 14. Data on accelerated stability of F5-1 (25±2 °C)**

| Test items | Acceptance criteria | Study time | | | |
|---|---|---|---|---|---|
| | | Day 0 | 1 month | 2 months | 3 months |
| Appearance (visual inspection) | Should be clear to slightly opalescent, colorless or nearly colorless liquid | Clear, colorless liquid | Clear, colorless liquid | Clear, colorless liquid | Clear, colorless liquid |
| GLP-1 activity (cell bioassay) | Should be 60-140% of the control | 113 | 95 | 97 | 99 |
| Glucagon activity (cell bioassay) | Should be 60-140% of the control | 71 | 87 | 60 | 75 |
| Main peak purity (RP-HPLC method) | Should be ≥ 92.0% | 97.9 | 97.2 | 96.1 | 95.7 |
| Related substances (RP-HPLC method) | Maximum unspecific impurities: should be ≤ 4.0% | 0.6 | 0.7 | 0.8 | 0.9 |
| | Total impurities: should be ≤ 8.0% | 2.1 | 2.8 | 3.9 | 4.3 |
| Monomer/polyme r (SEC-HPLC method) | Monomer: should be ≥ 97.0% | 99.4 | 99.3 | 99.3 | 99.2 |
| | Polymer: should be ≤ 3.0% | 0.3 | 0.5 | 0.6 | 0.6 |
| Visible particles (test for visible particles) | Should comply with specification | Comply with specification | N/A | N/A | N/A |
| pH value (method for determining pH) | Should be 7.5-8.5 | 7.7 | 7.7 | 7.6 | 7.7 |
| Insoluble particles (light blockage) | The number of particles with the diameter ≥ 10 µm in each vial should be ≤ 6000 | 1278 | N/A | N/A | N/A |
| | The number of particles with the diameter ≥ 25 µm in each vial should be ≤ 600 | 6 | N/A | N/A | N/A |
| Content determination (RP-HPLC method) | Should be 90.0-110.0% of the labeled amount | 96.5 | 96.1 | 94.2 | 93.0 |

**Table 15. Data on accelerated stability of F5-2 (25±2 °C)**

| Test items | Acceptance criteria | Study time | | | |
|---|---|---|---|---|---|
| | | Day 0 | 1 month | 2 months | 3 months |
| Appearance (visual inspection) | Should be clear to slightly opalescent, colorless or nearly colorless liquid | Clear, colorless liquid | Clear, colorless liquid | Clear, colorless liquid | Clear, colorless liquid |
| GLP-1 activity (cell bioassay) | Should be 60-140% of the control | 96 | 99 | 100 | 99 |
| Glucagon activity (cell bioassay) | Should be 60-140% of the control | 93 | 85 | 87 | 93 |
| Main peak purity (RP-HPLC method) | Should be ≥ 92.0% | 98.0 | 97.1 | 96.2 | 95.8 |
| Related substances (RP-HPLC method) | Maximum unspecific impurities: should be ≤ 4.0% | 0.6 | 0.7 | 0.8 | 0.9 |
| | Total impurities: should be ≤ 8.0% | 2.0 | 2.9 | 3.8 | 4.2 |
| Monomer/polymer (SEC-HPLC method) | Monomer: should be ≥ 97.0% | 99.5 | 99.4 | 99.3 | 99.2 |
| | Polymer: should be ≤ 3.0% | 0.3 | 0.5 | 0.6 | 0.6 |
| Visible particles (test for visible particles) | Should comply with specification | Comply with specification | N/A | N/A | N/A |
| pH value (method for determining pH) | Should be 7.5-8.5 | 7.7 | 7.7 | 7.6 | 7.7 |
| Insoluble particles (light blockage) | The number of particles with the diameter ≥ 10 µm in each vial should be ≤ 6000 | 1312 | N/A | N/A | N/A |
| | The number of particles with the diameter ≥ 25 µm in each vial should be ≤ 600 | 11 | N/A | N/A | N/A |
| Content determination (RP-HPLC method) | Should be 90.0-110.0% of the labeled amount | 97.3 | 96.9 | 96.7 | 94.2 |

The data in Tables 16-17 show that the F5-1 and F5-2 samples meet the quality standard in all the test items after storage at 5±3 °C for 6 months and showed no significant changes.

**Table 16. Data on long-term stability of F5-1 (5±3 °C)**

| Test items | Acceptance criteria | Study time | | |
|---|---|---|---|---|
| | | 0 months | 3 months | 6 months |
| Appearance (visual inspection) | Should be clear to slightly opalescent, colorless or nearly colorless liquid | Clear, colorless liquid | Clear, colorless liquid | Clear, colorless liquid |
| GLP-1 activity (cell bioassay) | Should be 60-140% of the control | 113 | 96 | 98 |
| Glucagon activity (cell bioassay) | Should be 60-140% of the control | 71 | 96 | 88 |
| Main peak purity (RP-HPLC method) | Should be ≥ 92.0% | 97.9 | 98.0 | 97.7 |
| Related substances (RP-HPLC method) | Maximum unspecific impurities: should be ≤ 4.0% | 0.6 | 0.7 | 0.7 |
| | Total impurities: should be ≤ 8.0% | 2.1 | 2.0 | 2.3 |
| Monomer/polymer (SEC-HPLC method) | Monomer: should be ≥ 97.0% | 99.4 | 99.3 | 99.4 |
| | Polymer: should be ≤ 3.0% | 0.3 | 0.4 | 0.4 |
| pH value (method for determining pH) | Should be 7.5-8.5 | 7.7 | 7.7 | 7.7 |
| Content determination (RP-HPLC method) | Should be 90.0-110.0% of the labeled amount | 96.5 | 94.0 | 95.6 |

**Table 17. Data on long-term stability of F5-2 (5±3 °C)**

| Test items | Acceptance criteria | Study time | | |
|---|---|---|---|---|
| | | 0 months | 3 months | 6 months |
| Appearance (visual inspection) | Should be clear to slightly opalescent, colorless or nearly colorless liquid | Clear, colorless liquid | Clear, colorless liquid | Clear, colorless liquid |
| GLP-1 activity (cell bioassay) | Should be 60-140% of the control | 96 | 98 | 101 |
| Glucagon activity (cell bioassay) | Should be 60-140% of the control | 93 | 92 | 84 |
| Main peak purity (RP-HPLC method) | Should be ≥ 92.0% | 98.0 | 98.0 | 97.7 |
| Related substances (RP-HPLC method) | Maximum unspecific impurities: should be ≤ 4.0% | 0.6 | 0.7 | 0.7 |
| | Total impurities: should be ≤ 8.0% | 2.0 | 2.0 | 2.3 |
| Monomer/polymer (SEC-HPLC method) | Monomer: should be ≥ 97.0% | 99.5 | 99.4 | 99.5 |
| | Polymer: should be ≤ 3.0% | 0.3 | 0.4 | 0.4 |
| pH value (method for determining pH) | Should be 7.5-8.5 | 7.7 | 7.7 | 7.6 |
| Content determination (RP-HPLC method) | Should be 90.0-110.0% of the labeled amount | 97.3 | 95.6 | 96.1 |

II. The F5-3 sample prepared in Example 3 was subjected to study for forced stability, accelerated stability, long-term stability, shaking stability and illumination stability. The specific scheme is shown in Table 18.

**Table 18. Experimental conditions and sampling schedule**

| Name of experiment | Sample | Experimental conditions and sampling schedule | Test items |
|---|---|---|---|
| Experiment on forced stability | F5-3 | Stored at 40±2 °C, sampling at day 0, week 1, week 2 and month 1 | Appearance, visible particles, content (RP-HPLC method), insoluble particles (light blockage), purity (SEC-HPLC method and RP-HPLC method), charge variants (AEX method) and biological activity (cell-based method) |
| Experiment on accelerated stability | | Stored at 25±2 °C, sampling at day 0, week 2, month 1 and month 3 | |
| Experiment on long-term stability | | Stored at 5±3 °C, sampling at day 0, month 3 and month 6 | |
| Shaking experiment | | Room temperature, 650 r/min, in the dark; sampling at days 0, 1, 3 and 5 | |
| Illumination experiment | | Stored at 25±2 °C, 60±5% RH, 500±50 Lux, sampling at days 0, 1, 3, 5 and 10 | |

### Analysis of results

### 1. Appearance and visible particles

The F5-3 sample was acceptable in terms of appearance and visible particles after storage at 40±2 °C for 1 month, at 25±2 °C for 3 months and at 5±3 °C for 6 months. For the description of the appearance on day 0 and after storage for 1 month under forced condition, the sample was clear to slightly opalescent, colorless or nearly colorless liquid.

### 2. Content

The results of drug content are shown in Table 19. The results show that the drug content of each sample showed no significant change after storage at 40±2 °C for 1 month, at 25±2 °C for 3 months and at 5±3 °C for 6 months.

**Table 19. Results of drug content**

| Sample name | Day 0 | 40 °C±2 °C | | | 25 °C±2 °C | | | 5 °C±3 °C | |
|---|---|---|---|---|---|---|---|---|---|
| | | 1 week | 2 weeks | 1 month | 2 weeks | 1 month | 3 months | 3 months | 6 months |
| F5-3 | 5.9 | 5.6 | 5.7 | 5.8 | 5.8 | 6.0 | 5.8 | 6.0 | 6.2 |

### 3. Purity

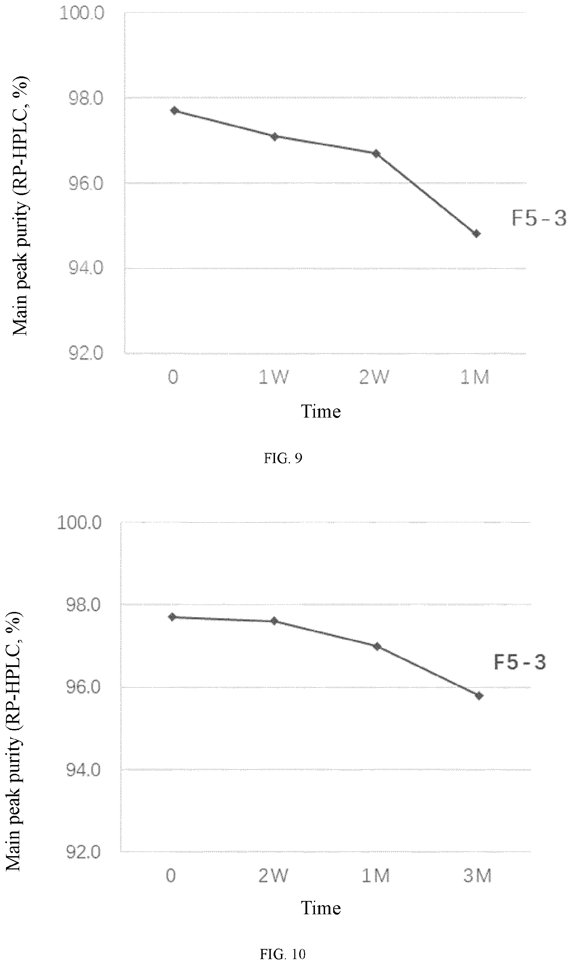

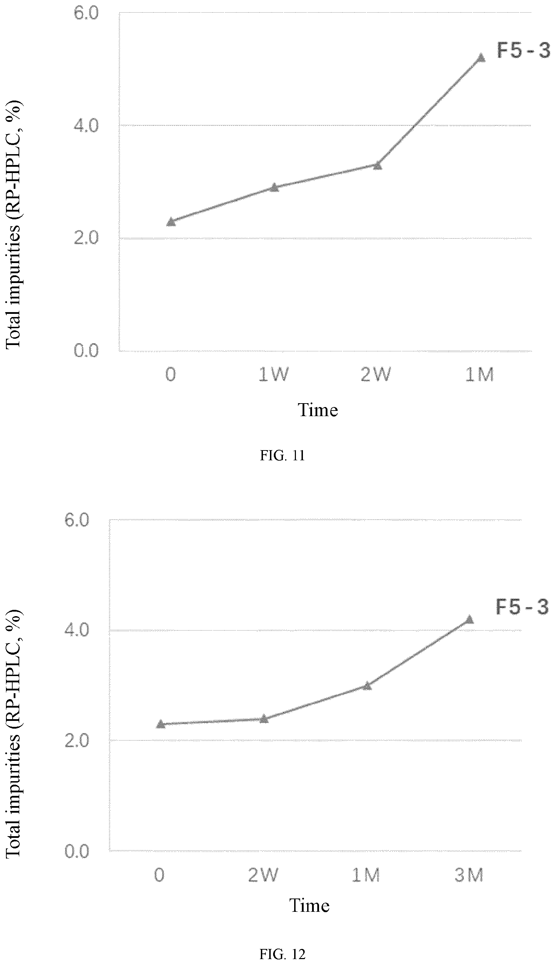

The results of purity are shown in Table 20. The trend of change is shown in FIGs. 9-12.

The results of monomer purity show that the purity of the F5-3 sample (SEC-HPLC method) showed no significant change after storage at 40±2 °C for 1 month, at 25±2 °C for 3 months and at 5±3 °C for 6 months.

**Table 20. Results of purity**

| Test items | Sample name | Day 0 | 40 °C±2 °C | | | 25 °C±2 °C | | | 5 °C±3 °C | |
|---|---|---|---|---|---|---|---|---|---|---|
| | | | 1 week | 2 weeks | 1 month | 2 weeks | 1 month | 3 months | 3 months | 6 months |
| Monomer purity (SEC-HPLC method, %) | F5-3 | 99.5 | 99.4 | 99.3 | 98.9 | 99.2 | 99.1 | 99.1 | 99.3 | 99.1 |
| Main peak purity (RP-HPLC method, %) | F5-3 | 97.7 | 97.1 | 96.7 | 94.8 | 97.6 | 97.0 | 95.8 | 97.6 | 97.7 |
| Total impurities (RP-HPLC method, %) | F5-3 | 2.3 | 2.9 | 3.3 | 5.2 | 2.4 | 3.0 | 4.2 | 2.4 | 2.3 |
| Maximum single impurity (RP-HPLC method, %) | F5-3 | 0.7 | 0.6 | 0.8 | 1.1 | 0.7 | 0.7 | 0.8 | 0.6 | 0.5 |

The results of main peak purity show that the main peak purity of the F5-3 sample showed changes of different degrees after storage at 40±2 °C for 1 month, at 25±2 °C for 3 months and at 5±3 °C for 6 months. After storage at 40±2 °C for 1 month, the main peak purity of the F5-3 sample was decreased significantly, but the change was acceptable in terms of the quality standards. Compared with values on day 0, the main peak purity of the F5-3 sample was decreased by 2.9%, the total impurity was increased by 2.9%, and the maximum single impurity showed no significant change and was increased only by 0.4%. After storage at 25±2 °C for 3 months, the main peak purity of the F5-3 sample was decreased by 1.9%, the total impurity was increased by 1.9%, and the maximum single impurity showed no significant change and was increased only by 0.1%. After storage at 5±3 °C for 6 months, the F5-3 sample showed no significant changes in terms of the main peak purity, the total impurity and the maximum single impurity.

The results of the purity experiments above indicate that the formula F5 is stable.

### 4. Charge variants

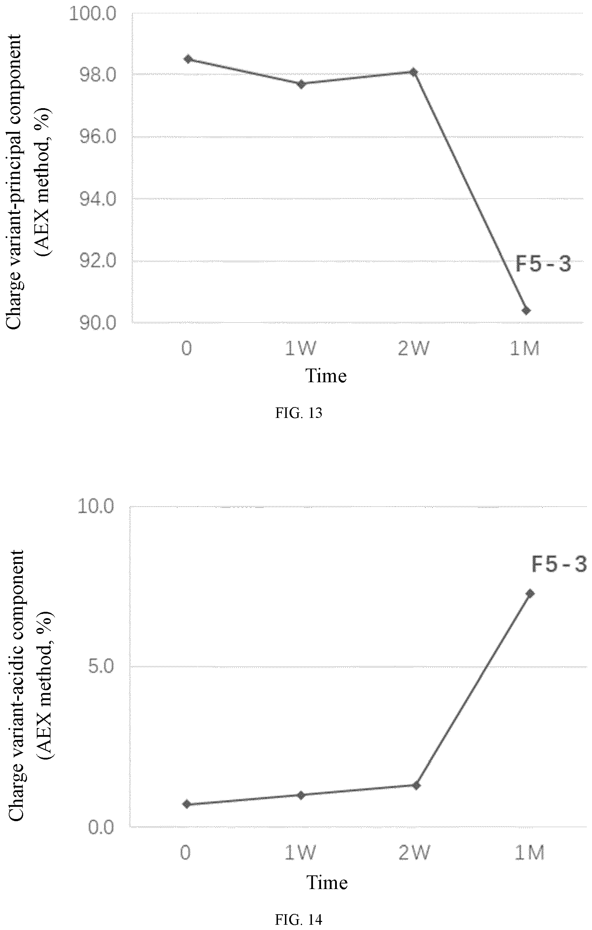

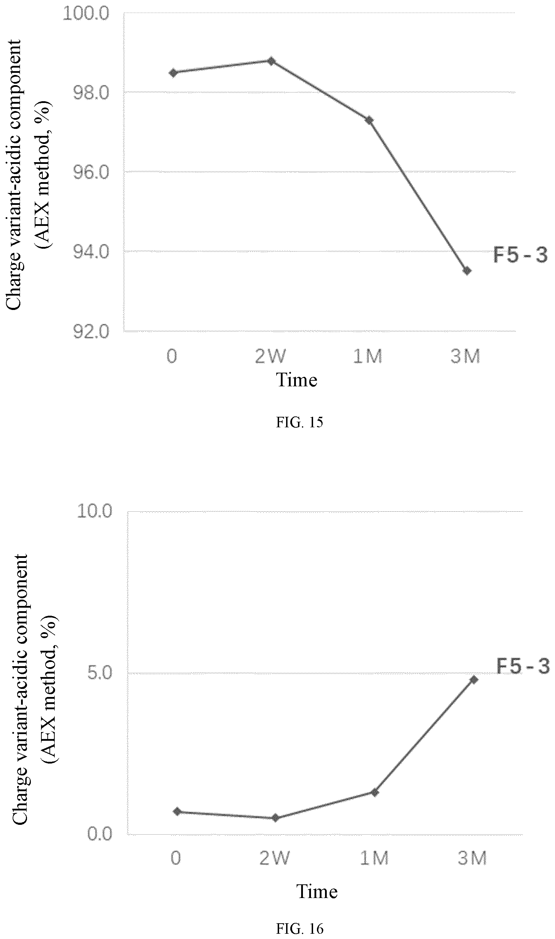

The results of charge variants are shown in Table 21. The trend of change is shown in FIGs. 13-16.

The results show that after storage at 40±2 °C for 1 month, the principal component and the acidic component of the charge variants of the F5-3 sample showed significant changes. Compared with values on day 0, the principal component of the F5-3 sample was decreased by 8.1%, and the acidic component was increased by 6.6%. After storage at 25±2 °C for 3 months, the principal component and the acidic component of the F5-3 sample showed significant changes, but the changes were acceptable in terms of the quality standards. Compared with values on day 0, the principal component of the F5-3 sample was decreased by 5.0%, and the acidic component was increased by 4.1%. After storage at 5±3 °C for 6 months, the principal component, the acidic component and the basic component of the charge variants of the F5-3 sample showed no significant changes.

**Table 21. Results of charge variants**

| Sample name | | Day 0 | 40 °C±2 °C | | | 25 °C±2 °C | | | 5 °C±3 °C | |
|---|---|---|---|---|---|---|---|---|---|---|
| | | | 1 week | 2 weeks | 1 month | 2 weeks | 1 month | 3 months | 3 months | 6 months |
| F5-3 | Acidic component | 0.7 | 1.0 | 1.3 | 7.3 | 0.5 | 1.3 | 4.8 | 0.4 | 0.7 |
| | Principal component | 98.5 | 97.7 | 98.1 | 90.4 | 98.8 | 97.3 | 93.5 | 98.5 | 98.2 |
| | Basic component | 0.7 | 1.3 | 0.7 | 2.3 | 0.7 | 1.4 | 1.7 | 1.1 | 1.1 |

### 5. Biological activity

The results of the biological activity are shown in Table 22. The results show that the biological activity (cell-based method) of the F5-3 sample was acceptable within the range of 60-140% after storage at 40±2 °C for 1 month, at 25±2 °C for 3 months and at 5±3 °C for 6 months.

**Table 22. Results of biological activity (cell-based method)**

| Test items | Sample name | Day 0 | 40 °C±2 °C | 25 °C±2 °C | | 5 °C±3 °C | |
|---|---|---|---|---|---|---|---|
| | | | 1 month | 1 month | 3 months | 3 months | 6 months |
| GLP-1 activity (cell-based method, %) | F5-3 | 105 | 88 | 94 | 111 | 110 | 90 |
| Glucagon activity (cell-based method, %) | F5-3 | 104 | 127 | 84 | 110 | 71 | 95 |

### 6. Insoluble particles

The results of insoluble particles are shown in Table 23. The results show that the F5-3 was acceptable in terms of insoluble particles after storage at 40±2 °C for 2 weeks and at 25±2 °C for 1 month.

**Table 23. Results of insoluble particles (light blockage)**

| Sample name | Test items | | Day 0 | 40 °C±2 °C | 25 °C±2 °C |
|---|---|---|---|---|---|
| | | | | 2 weeks | 1 month |
| F5-3 | Insoluble particles (light blockage, particle/mL) | ≥10 µm | 31 | 75 | 280 |
| | | ≥25 µm | 0 | 1 | 0 |

### 4. Results of shaking experiment

The results of the shaking experiment are shown in Table 24. The results show that after being shaken at 650 r/min for 5 days at room temperature and in the dark, the F5-3 was acceptable in terms of appearance and visible particles, and no significant changes were found for purity, charge variants and biological activity.

**Table 24. Results of shaking experiment**

| Sample name | F5-3 | | | | |
|---|---|---|---|---|---|
| Test items | Time (day) | | | | |
| | 0 | 1 | 3 | 5 | |
| Appearance (visual inspection) | Acceptable | Acceptable | Acceptable | Acceptable | |
| Visible particles (test for visible particles) | Acceptable | Acceptable | Acceptable | Acceptable | |

| 0 | 1 | 3 | 5 | | |
|---|---|---|---|---|---|
| Monomer purity (SEC-HPLC method, %) | | 99.5 | 99.5 | 99.5 | 99.5 |
| Purity (RP-HPLC method, %) | Main peak purity | 97.7 | 97.7 | 97.8 | 97.4 |
| | Total impurities | 2.3 | 2.3 | 2.2 | 2.6 |
| | Maximum single impurity | 0.7 | 0.7 | 0.7 | 0.7 |
| Charge variants (AEX method, %) | Acidic component | 0.7 | 0.3 | 0.3 | 0.4 |
| | Principal component | 98.5 | 98.6 | 98.5 | 98.5 |
| | Basic component | 0.7 | 1.0 | 1.2 | 1.1 |
| Biological activity (cell-based method, %) | GLP-1 | 105 | / | / | 91 |
| | Glucagon | 104 | / | / | 126 |

| | | | | | |
|---|---|---|---|---|---|
| Note: "/" indicates not set. | | | | | |

### (5) Results of illumination experiment

The results of the illumination experiment are shown in Table 25. The results show that after storage for 10 days under conditions of 25±2 °C, 60±5% RH and 500±50 Lux, the F5-3 samples were acceptable in terms of appearance and visible particles, no significant changes were found for purity, charge variants and biological activity, and no significant differences were present between the F5-3 samples.

**Table 25. Results of illumination experiment**

| Sample name | | F5-3 | | | | |
|---|---|---|---|---|---|---|
| Test items | | Time (day) | | | | |
| | | 0 | 1 | 3 | 5 | 10 |
| Appearance (visual inspection) | | Acceptable | Acceptable | Acceptable | Acceptable | Acceptable |
| Visible particles (test for visible particles) | | Acceptable | Acceptable | Acceptable | Acceptable | Acceptable |
| Purity (SEC-HPLC method, %) | | 99.5 | 99.5 | 99.5 | 99.4 | 99.3 |
| Purity (RP-HPLC method, %) | Main peak purity | 97.7 | 97.7 | 97.6 | 97.5 | 98.0 |
| | Total impurities | 2.3 | 2.3 | 2.4 | 2.5 | 2.0 |
| | Maximum single impurity | 0.7 | 0.7 | 0.7 | 0.7 | 0.6 |
| Charge variants (AEX method, %) | Acidic component | 0.7 | 0.4 | 0.4 | 0.4 | 0.4 |
| | Principal component | 98.5 | 98.7 | 98.5 | 98.5 | 99.0 |
| | Basic component | 0.7 | 1.0 | 1.1 | 1.1 | 0.6 |
| Biological activity (cell-based method, %) | GLP-1 | 105 | / | / | / | 103 |
| | Glucagon | 104 | / | / | / | 83 |

| | | | | | | |
|---|---|---|---|---|---|---|
| Note: "/" indicates not set. | | | | | | |

III. The F5-4 and F5-5 samples prepared in Example 3 were subjected to study for forced stability and accelerated stability. The specific scheme is shown in Table 26.

**Table 26. Experimental conditions and sampling schedule**

| Name of experiment | Sample | Experimental conditions and sampling schedule | Test items |
|---|---|---|---|
| Experiment on forced stability | F5-4 and F5-5 | Stored at 40±2 °C, sampling at day 0, week 1, week 2 and month 1 | Appearance, visible particles, content (RP-HPLC method), insoluble particles (light blockage), purity (SEC-HPLC method and RP-HPLC method), charge variants (AEX method) and biological activity (cell-based method) |
| Experiment on accelerated stability | | Stored at 25±2 °C, sampling at day 0, week 2, month 1 and month 3 | |

### Analysis of results

The data in Tables 27-28 show that after storage at 40±2 °C for 1 month and at 25±2 °C for 3 months, the F5-4 and F5-5 samples showed no significant changes in test items of activity and insoluble particles; they showed changes in test items of content, purity and related substances, but the changes were acceptable in terms of the quality standards.

**Table 27. Data on stability of F5-4**

| Test items | | Acceptance criteria | Day 0 | 40 °C, 1 week | 40 °C, 2 weeks | 40 °C, 1 month | 25 °C, 1 month | 25 °C, 3 months |
|---|---|---|---|---|---|---|---|---|
| Content determination (RP-HPLC method) | | Should be 90.0-110.0% of the labeled amount | 96.0 | 100.9 | 87.6 | 86.8 | 94.7 | 92.7 |
| Purity | Monomer (SEC-HPLC method), % | ≥97.0% | 99.5 | 99.4 | 99.3 | 99.0 | 99.3 | 99.2 |
| | Main peak purity (RP-HPLC method), % | ≥92.0% | 98.0 | 96.7 | 95.4 | 92.6 | 96.6 | 94.6 |
| | Total impurities (RP-HPLC method), % | ≤8.0% | 2.0 | 3.3 | 4.6 | 7.4 | 3.4 | 5.4 |
| | Maximum unspecific impurities (RP-HPLC method), % | ≤4.0% | 0.6 | 0.7 | 0.8 | 1.6 | 0.7 | 0.9 |
| Charge variants (AEX method) | Acidic component, % | N/A | 0.7 | 1.6 | 2.3 | 9.3 | 1.9 | 6.7 |
| | Principal component, % | N/A | 98.6 | 97.0 | 95.8 | 88.0 | 96.7 | 91.4 |
| | Basic component, % | N/A | 0.7 | 1.4 | 1.8 | 2.7 | 1.4 | 1.9 |
| Activity (cell bioassay) | GLP-1, % | 60%-140% | 83 | N/S | 110 | 113 | N/S | 85 |
| | GCGR, % | 60%-140% | 109 | N/S | 114 | 95 | N/S | 103 |
| Insoluble particles (light blockage) | ≥2 µm | N/A | 16 | N/S | 469 | 35 | N/S | 49 |
| | ≥5 µm | N/A | 8 | N/S | 97 | 4 | N/S | 7 |
| | ≥10 µm | ≤6000 | 1 | N/S | 15 | 1 | N/S | 3 |
| | ≥25 µm | ≤600 | 0 | N/S | 0 | 0 | N/S | 1 |

**Table 28. Data on stability of F5-5**

| Test items | | Acceptance criteria | Day 0 | 40 °C, 1 week | 40 °C, 2 weeks | 40 °C, 1 month | 25 °C, 1 month | 25 °C, 3 months |
|---|---|---|---|---|---|---|---|---|
| Content determination (RP-HPLC method) | | Should be 90.0-110.0% of the labeled amount | 95.8 | 92.0 | 88.2 | 87.6 | 90.2 | 96.0 |
| Purity | Monomer (SEC-HPLC method), % | ≥97.0% | 99.4 | 99.5 | 99.3 | 99.0 | 99.4 | 99.2 |
| | Main peak purity (RP-HPLC method), % | ≥92.0% | 98.1 | 96.7 | 95.4 | 92.4 | 96.7 | 94.8 |
| | Total impurities (RP-HPLC method), % | ≤8.0% | 1.9 | 3.3 | 4.6 | 7.6 | 3.3 | 5.2 |
| | Maximum unspecific impurities (RP-HPLC method), % | ≤4.0% | 0.6 | 0.7 | 0.8 | 1.6 | 0.7 | 0.9 |
| Charge variants (AEX method) | Acidic component, % | N/A | 0.4 | 2.1 | 3.5 | 9.4 | 2.4 | 6.6 |
| | Principal component, % | N/A | 98.9 | 96.4 | 94.6 | 87.8 | 96.2 | 91.6 |
| | Basic component, % | N/A | 0.7 | 1.5 | 1.9 | 2.7 | 1.4 | 1.8 |
| Activity (cell bioassay) | GLP-1, % | 60%-140% | 109 | N/S | 82 | 109 | N/S | 81 |
| | GCGR, % | 60%-140% | 95 | N/S | 97 | 101 | N/S | 99 |
| Insoluble particles (light blockage) | ≥2 µm | N/A | 83 | N/S | 548 | 361 | N/S | 114 |
| | ≥5 µm | N/A | 7 | N/S | 57 | 83 | N/S | 28 |
| | ≥10 µm | ≤6000 | 0 | N/S | 3 | 13 | N/S | 8 |
| | ≥25 µm | ≤600 | 0 | N/S | 0 | 2 | N/S | 0 |

IV The F5-7 sample prepared in Example 3 was subjected to study for forced stability and accelerated stability. The specific scheme is shown in Table 29.

**Table 29. Experimental conditions and sampling schedule**

| Name of experiment | Sample | Experimental conditions and sampling schedule | Test items |
|---|---|---|---|
| Experiment on forced stability | F-7 | Stored at 40±2 °C, sampling at day 0, week 1, week 2 and month 1 | Appearance, visible particles, content (RP-HPLC method), purity (SEC-HPLC method and RP-HPLC method) and biological activity (cell-based method) |
| Experiment on accelerated stability | | Stored at 25±2 °C, sampling at day 0, week 2, month 1 and month 3 | |

### Analysis of results

The data in Table 30 show that after storage at 40±2 °C for 1 month and at 25±2 °C for 3 months, the F5-7 sample showed no significant change in test item of activity; it showed changes in test items of content, purity and related substances, but the changes were acceptable in terms of the quality standards.

**Table 30. Data on stability of F5-7**

| Test items | | Acceptance criteria | Day 0 | 40 °C, 1 week | 40 °C, 2 weeks | 40 °C, 1 month | 25 °C, 1 month | 25 °C, 3 months |
|---|---|---|---|---|---|---|---|---|
| Content determination (RP-HPLC method) | | Should be 90.0-110.0% of the labeled amount | 97.5 | 98.5 | 91.5 | 91.5 | 98.0 | 97.0 |
| Purity | Monomer (SEC-HPLC method), % | ≥97.0% | 99.7 | 99.5 | 99.4 | 99.2 | 99.6 | 99.3 |
| | Main peak purity (RP-HPLC method), % | ≥92.0% | 98.6 | 98.1 | 97.0 | 94.7 | 98.1 | 96.7 |
| | Total impurities (RP-HPLC method), % | ≤8.0% | 1.4 | 1.9 | 3.0 | 5.3 | 1.9 | 3.3 |
| | Maximum unspecific impurities (RP-HPLC method), % | ≤4.0% | 0.5 | 0.4 | 0.7 | 1.5 | 0.5 | 0.6 |
| Activity (cell bioassay) | GLP-1, % | 60%-140% | 110 | 104 | 130 | 87 | 90 | 99 |
| | GCGR, % | 60%-140% | 126 | 110 | 121 | 115 | 100 | 100 |
| Osmotic pressure (osmolarity assay, mOsmol/kg) | | 240∼340 mOsmol/kg | 289 | N/S | N/S | 290 | N/S | 288 |

Based on the experimental results above and the experience in developing formulas of formulations, finally F5 was selected as the formula of OXM3 formulation, and its composition is as follows: wherein the prescription comprises the following components: 3.0, 4.0, 6.0, 12, 15, 18 or 20 mg/mL OXM3, 1.21 mg/mL tromethamine (i.e., tris(hydroxymethyl)aminomethane, Tris), 23.0 mg/mL mannitol, 10.0 mg/mL propylene glycol, 0.05 mg/mL edetate disodium, pH 7.7.

## Claims

1. An OXM3 storage agent, comprising 0.5-5 mg/mL tromethamine, 0.1-100 mg/mL stabilizer, 0.01-5 mg/mL chelating agent and a solvent, wherein: the stabilizer comprises one or more of mannitol, propylene glycol, arginine, arginine hydrochloride, histidine and histidine hydrochloride, and preferably the stabilizer comprises mannitol and propylene glycol; the chelating agent comprises edetate disodium; the solvent comprises water.

2. The OXM3 storage agent according to claim 1, wherein: the storage agent comprises 1-3 mg/mL tromethamine, 10-66 mg/mL stabilizer, 0.03-1 mg/mL chelating agent and a solvent; preferably, the storage agent comprises 1.21 mg/mL tromethamine, 20-46 mg/mL stabilizer, 0.05-0.5 mg/mL chelating agent and a solvent.

3. The OXM3 storage agent according to claim 2, wherein: the storage agent consists of 1.21 mg/mL tromethamine, 46 mg/mL mannitol, 0.5 mg/mL edetate disodium and water as a solvent;
the storage agent consists of 1.21 mg/mL tromethamine, 20 mg/mL propylene glycol, 0.5 mg/mL edetate disodium and water as a solvent; or
the storage agent consists of 1.21 mg/mL tromethamine, 10 mg/mL propylene glycol, 23 mg/mL mannitol, 0.05 mg/mL edetate disodium and water as a solvent.

4. The OXM3 storage agent according to claim 2, further comprising a surfactant, preferably tween 80.

5. An OXM3 formulation, comprising the OXM3 storage agent according to any one of claims 1-4 and 1-100 mg/mL OXM3, wherein: preferably, the OXM3 has a concentration of 3-50 mg/mL; more preferably, the OXM3 has a concentration of 6-20 mg/mL; still preferably, the OXM3 has a concentration of 15-20 mg/mL; most preferably, the OXM3 has a concentration of 18 mg/mL.

6. The OXM3 formulation according to claim 5, wherein: the OXM3 formulation has a pH of 7-9; preferably, the OXM3 formulation has a pH of 7.5-8.5; more preferably, the OXM3 formulation has a pH of 7.7.

7. The OXM3 formulation according to claim 6, wherein: the OXM3 formulation consists of 8.37 mg/mL OXM3, 1.21 mg/mL tromethamine, 46 mg/mL mannitol, 0.5 mg/mL edetate disodium and water as a solvent, and the OXM3 formulation has a pH of 7.7;
the OXM3 formulation consists of 8.37 mg/mL OXM3, 1.21 mg/mL tromethamine, 20 mg/mL propylene glycol, 0.5 mg/mL edetate disodium and water as a solvent, and the OXM3 formulation has a pH of 7.7;
the OXM3 formulation consists of 3 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7;
the OXM3 formulation consists of 4 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7;
the OXM3 formulation consists of 6 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7;
the OXM3 formulation consists of 12 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7;
the OXM3 formulation consists of 15 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7;
the OXM3 formulation consists of 18 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7; or
the OXM3 formulation consists of 20 mg/mL OXM3, 1.21 mg/mL tromethamine, 10 mg/mL propylene glycol, 23 mg/mL mannitol, 0.05 mg/mL edetate disodium and water as a solvent, and the OXM3 formulation has a pH of 7.7.

8. A method for preparing the OXM3 formulation according to claim 6 or 7, comprising the steps of:
(1) mixing tromethamine, a stabilizer and a chelating agent to obtain an OXM3 storage agent;
(2) mixing OXM3 and the OXM3 storage agent obtained in step (1) to obtain a mixed solution; and
(3) adjusting a pH of the mixed solution and filtering to obtain the OXM3 formulation.

9. Use of the OXM3 storage agent according to any one of claims 1-4 in preserving OXM3.

10. A liquid formulation prepared from the storage agent according to any one of claims 5-7, wherein the liquid formulation is preferably a water injection.

11. A delivery device, comprising the OXM3 storage agent according to any one of claims 1-4, the OXM3 formulation according to any one of claims 5-7 or the liquid formulation according to claim 10, wherein:
the delivery device preferably comprises a container, a seal and an injection needle;
wherein:
the container is preferably a phial, a syringe or a vial;
the seal is preferably a sealing plug or a sealing ring;
the injection needle is preferably a water needle or a single needle-microneedle set;
more preferably, the delivery device is a pre-filled syringe.

12. A kit, comprising the OXM3 formulation according to any one of claims 5-7, the liquid formulation according to claim 10 or the delivery device according to claim 11.

13. Use of the OXM3 formulation according to any one of claims 5-7, the liquid formulation according to claim 10, the delivery device according to claim 11 or the kit according to claim 12 in preparing a product for treating or preventing a disease in a subject, wherein the disease is preferably obesity, diabetes mellitus and non-alcoholic steatohepatitis.
